# EUROPÄISCHE PATENTSCHRIFT

(11) **EP 0 939 617 B1**
(45) Veröffentlichungstag und Bekanntmachung des Hinweises auf die Patenterteilung: **23.03.2005**
(21) Anmeldenummer: 97912172.0
(22) Anmeldetag: 10.10.1997
(51) Int. Cl.: A61K 7/48, A61K 7/00

(54) **KOLLAGENFREIE KOSMETISCHE ZUBEREITUNGEN AUS VERNETZTEN CHITOSAN HYDROGELEN HERGESTELLT**
COLLAGENFREE COSMETIC PREPARATIONS OBTAINED FROM CROSS-LINKED CHITOSAN HYDROGELS
PREPARATIONS COSMETIQUES SANS COLLAGENE PRODUITES A PARTIR D'HYDROGELS DE CHITOSANE RETICULES

(30) Priorität: 18.10.1996 DE 19643066
(43) Veröffentlichungstag der Anmeldung: 08.09.1999
(73) Patentinhaber: Cognis Deutschland GmbH & Co. KG, 40589 Düsseldorf (DE)
(72) Erfinder: HEILEMANN, Andrea, D-89079 Ulm (DE); HOLZER, Josef, D-89257 Illertissen (DE); HORLACHER, Peter, D-89079 Ulm (DE); SANDER, Andreas, D-89257 Illertissen (DE); WACHTER, Rolf, D-40595 Düsseldorf (DE)
(86) Internationale Anmeldenummer: PCT/EP1997/005618
(87) Internationale Veröffentlichungsnummer: WO 1998/017245

(56) Entgegenhaltungen:
- WO-A-92/09635
- DE-A- 4 121 085
- GB-A- 2 296 250
- US-A- 5 322 935
- US-A- 5 420 197
- STN INTERNATIONAL; CHEMICAL ABSTRACTS. AN=115:287256, XP002057291 & JP 03 165 775 A (KATAKURA CHIKKARIN CO. LTD, JAPAN.)
- ROBERTS G A F ET AL: "CHITOSAN GELS, 3A) THE FORMATION OF GELS BY REACTION OF CHITOSAN WITH GLUTARALDEHYDE" MAKROMOLEKULARE CHEMIE, MACROMOLECULAR CHEMISTRY AND PHYSICS, Bd. 190, Nr. 5, 1.Mai 1989, Seiten 951-960, XP000132505

## Beschreibung

### Gebiet der Erfindung

Die Erfindung betrifft kollagenfreie kosmetische Zubereitungen, die man durch Vemetzung von kationischen Biopolymeren mit Diisocyanaten erhält sowie ein Verfahren zu ihrer Herstellung.

### Stand der Technik

Kosmetische Vliese werden als Feuchtigkeitsmasken für Gesicht und Hände verwendet. Üblicherweise werden diese Zubereitungen auf Basis von tierischem Kollagen hergestellt, indem man wäßrige Kollagensuspensionen auf einen pH-Wert im sauren Bereich einstellt und anschließend durch Gefriertrocknung entwässert. Im Zuge der anhaltenden Kritik an tierischen Produkten besteht im Markt ein wachsendes Bedürfnis nach Produkten, die ausschließlich unter Verwendung pflanzlicher oder mariner Rohstoffe hergestellt werden.

Aus der japanischen Patentanmeldung **JP-A2 Hei 6/048 917** (Nagawa) sind Schönheitspackungen mit Chitosan als aktiver Komponente sowie organischen Säuren und Kollagen als weiteren Bestandteilen bekannt. Gegenstand der japanischen Patentanmeldung **JP-A2 Hei 4/275 207** (Nitta Gelatin) sind feuchtigkeitsbindende Zusätze zu hautkosmetischen Mitteln, bei denen es sich um pulverförmige Mischungen von Chitosan und Kollagen handelt.

Aus den Veröffentlichungen US-A-5 322 935, JP-A-03165775 und GB-A-2 296 250 sind vernetzte Chitosan derivate bekannt.

Die Aufgabe der Erfindung hat somit darin bestanden, hautkosmetische Mittel zur Verfügung zu stellen, die einerseits frei von tierischem Kollagen sind und sich zum anderen zur Herstellung von Feuchtigkeitsmasken für Gesicht und Hände eignen.

### Beschreibung der Erfindung

Gegenstand der Erfindung sind kollagenfreie kosmetische Zubereitungen, die man erhält, indem man gequollene, wäßrige Suspensionen kationischer Biopolymere mit Glycerin und Diisocyanaten und/oder Dialdehyden vemetzt und anschließend entwässert.

Überraschenderweise wurde gefunden, daß mit den kationischen Biopolymeren wasserunlösliche, aber sehr gut mit Wasser benetzbare, elastische Fasergeflechte hergestellt werden können, die in ihren Eigenschaften den bekannten Kollagenschwämmen entsprechen. Die Erfindung schließt die Erkenntnis ein, daß mit anderen grundsätzlich in Betracht kommenden Ausgangsstoffen, wie beispielsweise pflanzlichen Proteinen oder faserbildenden Biopolymeren (z.B. Cellulose, Pektin) nur Produkte erhalten werden, die sich in der Anwendung als spröde erweisen oder in Wasser auflösen.

Ein weiterer Gegenstand der Erfindung betrifft ein Verfahren zur Herstellung von kollagenfreien kosmetischen Zubereitungen, bei dem man gequollene, wäßrige Suspensionen kationischer Biopolymere mit Glycerin und Diisocyanaten und/oder Dialdehyden versetzt und anschließend entwässert.

### Kationische Biopolymere

Kationische Biopolymere, wie z.B. Chitosane, werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

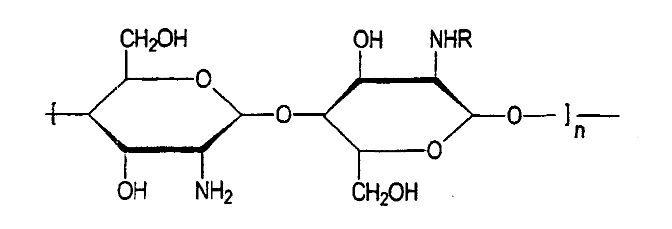

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332).** Übersichten zu diesem Thema sind auch beispielsweise von B.Gesslein et al. in **HAPPI 27, 57 (1990),** O.Skaugrud in **Drug Cosm.ind. 148, 24 (1991)** und E.Onsoyen et al. in **Seifen-Öle-Fette-Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 27 01266** bekannt.

### Vernetzungsmittel

Diisocyanate, die zur Vernetzung der kationischen Biopolymere in Betracht kommen, folgen vorzugsweise der Formel (I),

**O=CN-[X]-NC=O (I)**

in der X für einen linearen oder verzweigten, naphthenischen oder aromatischen Kohlenwasserstoffrest mit 1 bis 12 Kohlenstoffatomen steht. Vorzugsweise wird Hexamethylendiisocyanat als Vemetzungsmittel eingesetzt. Als Dialdehyde kommen Stoffe in Betracht, die der Formel (II) folgen,

**OHC-[Y]-CHO (II)**

in der Y für einen linearen oder verzweigten, naphthenischen oder aromatischen Kohlenwasserstoffrest mit 1 bis 12 Kohlenstoffatomen steht. Vorzugsweise wird Glutardialdehyd als Vemetzungsmittel eingesetzt.

Die Vernetzungsmittel können in Mengen von 0,5 bis 10, vorzugsweise 1 bis 8 und insbesondere 2 bis 5 Gew.-% - bezogen auf die Trockensubstanz der kationischen Biopolymere - eingesetzt werden.

Üblicherweise wird das Glycerin in Mengen von 0,1 bis 10, vorzugsweise 2 bis 8 Gew.-% - bezogen auf die Trockensubstanz des Chitosans eingesetzt.

### Herstellung der Zubereitungen

Üblicherweise werden wäßrige Lösungen bzw. Suspensionen der kationischen Biopolymere, vorzugsweise Chitosan, mit einem Trockensubstanzgehalt von 0,5 bis 3, vorzugsweise 1,8 bis 2,2 Gew.-% bei einem pH-Wert von 3,5 bis 6,0, vorzugsweise 5,0 bis 5,7 durch Zugabe von anorganischen oder organischen Säuren, vorzugsweise Salzsäure, hergestellt, wobei die Temperatur so gewählt werden sollte, daß sie die Quellung der Biopolymeren unterstützt. Üblicherweise liegt diese im Bereich von 20 bis 50 und vorzugsweise 35 bis 45°C. Die auf diesem Wege hergestellten Suspensionen enthalten neben den gelösten Biopolymeren auch gequollene ungelöste Teilchen. Die durch die genannten Bedingungen eingestellte Viskosität der Suspension kann die späteren mechanischen Eigenschaften der Vliese beeinflussen. Zur Erhaltung der Elastizität im getrockneten Zustand können den Suspensionen dann Polyole und weitere kosmetische Inhaltsstoffe zugesetzt werden. Für die machanischen Eigenschaften der Vliese hat es sich außerdem als vorteilhaft erwiesen, den Suspensionen natürliche Fasern, wie beispielsweise Lignin, Polyose, Pektin und insbesondere Cellulose, oder aber Synthesefasem wie beispielsweise Polyester, Polyamide oder deren Gemische in einer Menge von 1 bis 50, vorzugsweise 5 bis 10 Gew.-% zuzusetzen. Besonders empfehlenswert ist es, die Fasern vor der Homogenisierung der Lösung hinzuzugeben. Anschließend werden die Suspensionen homogenisiert, mit den Diisocyanaten und/oder Dialdehyden vemetzt und entwässert.

### Hilfs- und Zusatzstoffe

Die erfindungsgemäßen Zubereitungen können in untergeordneten Mengen mit den anderen Inhaltsstoffen kompatible Tenside enthalten. Typische Beispiele sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder vorzugsweise pflanzliche Proteinfettsäurekondensate.

Ferner können sie als weitere Hilfs- und Zusatzstoffe Ölkörper, Emulgatoren, Überfettungsmittel, Stabilisatoren, Wachse, Konsistenzgeber, Verdickungsmittel, Kationpolymere, Siliconverbindungen, biogene Wirkstoffe, Filmbildner, Konservierungsmittel, Hydrotrope, Solubilisatoren, UV-Adsorber, Farbund Duftstoffe und dergleichen enthalten.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, Guerbetcarbonate, Dialkylether, Siliconöle und/oder aliphatische bzw. naphthenische Kohlenwasserstoffe in Betracht.

Als Emulgatoren kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmonound -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte VinylpyrrolidonNinylimidazol-Polymere wie z.B. Luviquat® (BASF AG, Ludwigshafen/ FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quatemierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als Kon**servierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 0,1 bis 10, vorzugsweise 0, 5 bis 5 Gew.-% - bezogen auf die Trockensubstanz der kationischen Biopolymeren - betragen. Die Mittel können den Suspensionen zugegeben werden, es ist jedoch ebenfalls möglich, die fertigen Masken vor oder während der Anwendung mit diesen Stoffen zu befeuchten..

### Beispiele

### Beispiel 1:

In einer 2-I-Rührapparatur wurden 1960 ml Wasser vorgelegt und auf 40°C erhitzt und mit 40 g Chitosan (Hydagen® CMFP, Henkel KGaA, Düsseldorf/FRG) versetzt. Der pH-Wert der Mischung wurde durch Zugabe von Salzsäure auf 5,5 eingestellt. Anschließend wurden 2 g (5 Gew.-% bezogen auf Trockensubstanz) Glycerin hinzugegeben und die Mischung im Ultraturrax homogenisiert. Danach wurden 0,8 g (2 Gew.-% bezogen auf Trockensubstanz) Hexamethylendiisocyanat vorsichtig unterge-rührt. Nach der Vernetzung wurde die Suspension als Block eingefroren und anschließend lyophilisiert. Durch Spalten des entwässerten Blockes auf die gewünschte Dicke wurden elastische, wasserunlösliche Vliese erhalten, die sich bei Anfeuchten wie Schwämme verhielten.

### Beispiel 2:

In einer 2-I-Rührapparatur wurden 1960 ml Wasser vorgelegt und auf 40°C erhitzt und mit 40 g Chitosan (Hydagen® CMFP, Henkel KGaA, Düsseldorf/FRG) versetzt. Der pH-Wert der Mischung wurde durch Zugabe von Salzsäure auf 5,5 eingestellt. Anschließend wurden 2 g (5 Gew.-% bezogen auf Trockensubstanz) Glycerin und 2 g (5 Gew.-% bezogen auf Trockensubstanz) Cellulosefasern hinzugegeben und die Mischung im Ultraturrax homogenisiert. Danach wurden 0,8 g (2 Gew.-% bezogen auf Trockensubstanz) Hexamethylendiisocyanat vorsichtig unterge-rührt. Nach der Vemetzung wurde die Suspension als Block eingefroren und anschließend lyophilisiert. Durch Spalten des entwässerten Blockes auf die gewünschte Dicke wurden elastische, wasserunlösliche Vliese erhalten, die sich bei Anfeuchten wie Schwämme verhielten.

## Patentansprüche

1. Kollagenfreie kosmetische Zubereitungen, dadurch erhältlich, daß man gequollene wäßrige Suspensionen kationischer Biopolymere mit (a) Glycerin und (b) Diisocyanaten und/oder Dialdehyden vemetzt und anschließend entwässert.

2. Verfahren zur Herstellung von kollagenfreien kosmetischen Zubereitungen, bei dem man gequollene wäßrige Suspensionen kationischer Biopolymere (a) Glycerin und (b) mit Diisocyanaten und/oder Dialdehyden vemetzt und anschließend entwässert.

3. Verfahren nach Anspruch 2, **dadurch gekennzeichnet, daß** man als kationische Biopolymere Chitosane einsetzt.

4. Verfahren nach den Ansprüchen 2 und 3, **dadurch gekennzeichnet, daß** man Diisocyanate der Formel (I) einsetzt,
**O=CN-[X]-NC=O (I)**
in der X für einen linearen oder verzweigten, naphthenischen oder aromatischen Kohlenwasserstoffrest mit 1 bis 12 Kohlenstoffatomen steht.

5. Verfahren nach den Ansprüchen 2 bis 4, **dadurch gekennzeichnet, daß** man Dialdehyde der Formel (11) einsetzt,
**OHC-[Y]-CHO (II)**
in der Y für einen linearen oder verzweigten, naphthenischen oder aromatischen Kohlenwasserstoffrest mit 1 bis 12 Kohlenstoffatomen steht.

6. Verfahren nach den Ansprüchen 2 bis 5, **dadurch gekennzeichnet, daß** man als Vemetzungsmittel Hexamethylendiisocyanat und/oder Glutardialdehyd einsetzt.

7. Verfahren nach den Ansprüchen 2 bis 6, **dadurch gekennzeichnet, daß** man die Vemetzungsmittel in Mengen von 0,5 bis 10 Gew.-% - bezogen auf die Trockensubstanz der kationischen Biopolymere - einsetzt.

8. Verfahren nach den Ansprüchen 2 bis 7, **dadurch gekennzeichnet, daß** man Glycerin in Mengen von 0,1 bis 10 Gew.-% - bezogen auf die Trockensubstanz der kationischen Biopolymere - einsetzt.

9. Verfahren nach den Ansprüchen 2 bis 8, **dadurch gekennzeichnet, daß** man natürliche und/oder synthetische Fasern mitverwendet.

10. Verfahren nach Anspruch 9, **dadurch gekennzeichnet, daß** man die Fasern in Mengen von 1 bis 50 Gew.-% - bezogen auf die Trockensubstanz der kationischen Biopolymere - einsetzt.

11. Verfahren nach den Ansprüchen 2 bis 10, **dadurch gekennzeichnet, daß** man die Zubereitungen durch Gefriertrocknung entwässert.

## Claims

1. Collagen-free cosmetic preparations obtainable by crosslinking swollen aqueous suspensions of cationic biopolymers with (a) glycerol and (b) diisocyanates and/or dialdehydes and then removing the water present.

2. A process for the production of collagen-free cosmetic preparations in which swollen aqueous suspensions of cationic biopolymers are crosslinked with (a) glycerol and (b) with diisocyanates and/or dialdehydes and then freed from water.

3. A process as claimed in claim 2, **characterized in that** chitosans are used as the cationic biopolymers.

4. A process as claimed in claims 2 and 3, **characterized in that** diisocyanates corresponding to formula (I):
O=CN-[X]-NC=O (I)
in which X is a linear or branched, naphthenic or aromatic hydrocarbon radical containing 1 to 12 carbon atoms, are used.

5. A process as claimed in claims 2 to 4, **characterized in that** dialdehydes corresponding to formula (II):
OHC-[Y]-CHO (II)
in which Y is a linear or branched, naphthenic or aromatic hydrocarbon radical containing 1 to 12 carbon atoms, are used.

6. A process as claimed in claims 2 to 5, **characterized in that** hexamethylene diisocyanate and/or glutardialdehyde is/are used as the crosslinking agent(s).

7. A process as claimed in claims 2 to 6, **characterized in that** the crosslinking agents are used in quantities of 0.5 to 10% by weight, based on the dry matter content of the cationic biopolymers.

8. A process as claimed in claims 2 to 7, **characterized in that** glycerol is used in quantities of 0.1 to 10% by weight, based on the dry matter content of the cationic biopolymers.

9. A process as claimed in claims 2 to 8, **characterized in that** natural and/or synthetic fibers are used.

10. A process as claimed in claim 9, **characterized in that** the fibers are used in quantities of 1 to 50% by weight, based on the dry matter content of the cationic biopolymers.

11. A process as claimed in claims 2 to 10, **characterized in that** the preparations are freed from water by freeze drying.

## Revendications

1. Préparations cosmétiques sans collagène pouvant être obtenues en réticulant des suspensions aqueuses gonflées de biopolymères cationiques avec (a) du glycérol et (b) des diisocyanates et/ou des dialdéhydes puis en les déshydratant.

2. Procédé de fabrication de préparations cosmétiques sans collagène, selon lequel on réticule des suspensions aqueuses gonflées de biopolymères cationiques avec (a) du glycérol et (b) des diisocyanates et/ou des dialdéhydes puis on les déshydrate.

3. Procédé selon la revendication 2,
**caractérisé en ce que**
comme biopolymères cationiques on utilise des chitosanes.

4. Procédé selon les revendications 2 et 3,
**caractérisé en ce qu'**
on utilise des diisocyanates répondant à la formule (1)
O=CN-[X]-NC=O (I)
dans laquelle X représente un reste d'hydrocarbure naphténique ou aromatique linéaire ou ramifié, ayant 1 à 12 atomes de carbone.

5. Procédé selon les revendications 2 à 4,
**caractérisé en ce qu'**
on utilise des dialdéhydes répondant à la formule (II)
OHC-[Y]-CHO (II)
dans laquelle Y représente un reste d'hydrocarbure naphténique ou aromatique linéaire ou ramifié, ayant 1 à 12 atomes de carbone.

6. Procédé selon les revendications 2 à 5,
**caractérisé en ce que**
comme agent de réticulation on utilise de l'hexaméthylène diisocyanate et/ou du glutardialotéhyde.

7. Procédé selon les revendications 2 à 6,
**caractérisé en ce qu'**
on utilise l'agent de réticulation en quantités de 0,5 à 10 % en poids - rapportées à la matière sèche des biopolymères cationiques.

8. Procédé selon les revendications 2 à 7,
**caractérisé en ce qu'**
on utilise le glycérol en quantités de 0,1 à 10 % en poids - rapportées à la matière sèche des biopolymères cationiques.

9. Procédé selon les revendications 2 à 8,
**caractérisé en ce qu'**
on utilise simultanément des fibres naturelles et/ou synthétiques.

10. Procédé selon la revendication 9,
**caractérisé en ce qu'**
on utilise les fibres en quantités de 1 à 50 % en poids - rapportées à la matière sèche des biopolymères cationiques.

11. Procédé selon les revendications 2 à 10,
**caractérisé en ce qu'**
on déshydrate les préparations par lyophilisation.
